(19) Europäisches Patentamt European Patent Office Office européen des brevets

(11) **EP 4 477 621 A1**

(12) **EUROPEAN PATENT APPLICATION**
published in accordance with Art. 153(4) EPC

(43) Date of publication:
**18.12.2024  Bulletin 2024/51**

(21) Application number: **23803250.2**

(22) Date of filing: **23.03.2023**

(51) International Patent Classification (IPC):
**C01B 33/02** (2006.01)       **A61K 8/85** (2006.01)
**C09C 1/28** (2006.01)       **C09C 1/62** (2006.01)

(52) Cooperative Patent Classification (CPC):
**A61K 8/85; C01B 33/02; C09C 1/28; C09C 1/62; C09C 3/10**

(86) International application number:
**PCT/JP2023/011493**

(87) International publication number:
**WO 2023/218767 (16.11.2023 Gazette 2023/46)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:  **10.05.2022  JP 2022077669**

(71) Applicant: **Oike & Co., Ltd.**
**Kyoto-shi, Kyoto 600-8461 (JP)**

(72) Inventor: **SATO, Teiko**
**Kyoto-shi**
**Kyoto 601-8123 (JP)**

(74) Representative: **Bryn Aarflot AS**
**Patent**
**Stortingsgata 8**
**0161 Oslo (NO)**

(54) **METALLIC COLOR FLAKE POWDER, METALLIC COLOR COATING MATERIAL, METALLIC COLOR RESIN PELLETS, COSMETIC, CONTAINER, INTERIOR AND EXTERIOR MEMBERS FOR VEHICLES, AND METHOD FOR PRODUCING METALLIC COLOR FLAKE POWDER**

(57)    Provided is a flake-shaped metallic color powder having a silicon layer containing silicon and a translucent resin layer provided on both surfaces of the silicon layer, wherein a thickness of the silicon layer is 50 nm or more.

**EP 4 477 621 A1**

**Description**

TECHNICAL FIELD

The present invention relates to a flake-shaped metallic color powder, a metallic color paint, a metallic color resin pellet, a cosmetic product, a vessel, an interior/exterior member for vehicle, and a producing method of a flake-shaped metallic color powder. In more detail, the present invention relates to a flake-shaped metallic color powder, a metallic color paint, a metallic color resin pellet, a cosmetic product, a vessel, an interior/exterior member for vehicle, which can realize metallic color developments of various colors in a single layer, and a producing method of a flake-shaped metallic color powder.

BACKGROUND ART

Conventionally, a pigment for imparting a metallic color to various base materials has been considered. Patent Document 1 discloses a golden interference pigment that exhibits a golden interference color by providing at least one layer of $Fe_2O_3$ and the like on a flake-shaped base material having a green inherent interference color of $Al_2O_3$ and the like.

PRIOR ART DOCUMENT

Patent Document

Patent Document 1: JP 2015-532676 A

SUMMARY OF THE INVENTION

However, the golden interference pigment described in Patent Document 1 is a pigment that exhibits a golden color due to a so-called structural color (interference color). Therefore, the pigment described in Patent Document 1 cannot realize a golden color development unless two or more metal oxide layers having different refractive indices are laminated. Therefore, in such a metallic color pigment with a structural color, a pigment structure becomes complicated with lamination of a plurality of metal oxide layers, and the producing process also becomes complicated.

The present invention has been made in consideration of such conventional inventions, and it is an object of the present invention to provide a flake-shaped metallic color powder, a metallic color paint, a metallic color resin pellet, a cosmetic product, a vessel, an interior/exterior member for vehicle, which can realize metallic color developments of various colors in a single layer, and a producing method of a flake-shaped metallic color powder.

As a result of intensive studies, the present inventor has found that a flake-shaped metallic color powder in which a translucent resin layer is formed on both surfaces of a silicone layer and a thickness of the silicone layer is adjusted can solve the above-described problems, and completed the present invention. That is, the flake-shaped metallic color powder, the metallic color paint, the metallic color resin pellet, the cosmetic product, the vessel, the interior/exterior member for vehicle, and the producing method of the flake-shaped metallic color powder, of the present invention, mainly include the following configurations.

That is, the flake-shaped metallic color powder according to one embodiment of the present invention that solves the above-described problems is a flake-shaped metallic color powder having a silicon layer containing silicon and a translucent resin layer provided on both surfaces of the silicon layer, wherein a thickness of the silicon layer is 50 nm or more.

The metallic color paint according to one embodiment of the present invention that solves the above-described problems is a metallic color paint that contains the above-described flake-shaped metallic color powder, resin, and a solvent.

According to such a configuration, the metallic color paint can realize metallic color developments of various colors.

The metallic color resin pellet according to one embodiment of the present invention that solves the above-described problems is a metallic color resin pellet that contains the above-described flake metallic color powder and resin.

The cosmetic product according to one embodiment of the present invention that solves the above-described problems is a metallic color product that contains the above-described flake-shaped metallic color powder.

The method of producing a flake-shaped metallic color powder according to one embodiment of the present invention that solves the above-described problems is a method of producing a flake-shaped metallic color powder, the method comprising a laminated body-forming step and a peeling and pulverizing step, wherein the laminated body-forming step is a step of forming a laminated body on a base material, the step comprising a preparation step of preparing the base material, a first translucent resin layer-forming step of forming a first translucent resin layer on the base material, a silicon layer-forming step of forming a silicon layer containing silicon on the first translucent resin layer, and a second translucent resin layer-forming step of forming a second translucent resin layer on the silicon layer, and wherein the peeling and pulverizing step has a peeling step of peeling the laminated body from the base material and a pulverizing step of pulverizing the peeled laminated body to obtain a flake-shaped metallic color powder.

EMBODIMENT FOR CARRYING OUT THE INVENTION

<Flake-shaped metallic color powder>

The flake-shaped metallic color powder according to one embodiment of the present invention has a silicon layer containing silicon, and a translucent resin layer provided on both surfaces of the silicon layer. The silicon layer has a thickness of 50 nm or more. Each component will be described below.

(Silicon layer)

The silicon layer contains silicon (elementary silicon). A purity of the silicon is not particularly limited. By way of an example, the purity of silicon is preferably 95% by mass or more, more preferably 99% by mass or more, and further preferably 99.9% by mass or more. When the purity of silicon is within the above-described ranges, the flake-shaped metallic color powder easily realizes various metallic color developments.

The silicon layer may contain other components as appropriate depending on the purpose, as long as the effects of the present embodiment are exhibited. Moreover, the silicon layer may contain unavoidable impurities.

A thickness of the silicon layer may be 50 nm or more. Moreover, the thickness of the silicon layer is preferably 1 $\mu$m or less, more preferably 500 nm or less, and further preferably 300 nm or less. When the thickness of the silicon layer is less than 50 nm, the flake-shaped metallic color powder is less likely to realize a metallic color development. Moreover, when the thickness of the silicon layer is 1 $\mu$m or less, the flake-shaped metallic color powder easily obtains a desired color appearance. Besides, a method of measuring a thickness of the silicon layer is not particularly limited. By way of an example, the thickness of the silicon layer can be obtained by measuring a physical thickness with a cross-sectional SEM (S-4700, manufactured by Hitachi High-Tech Corporation).

(Translucent resin layer)

The translucent resin layer is a layer provided on both surfaces of the silicon layer. That is, the flake-shaped metallic color powder is a laminated body of a first translucent resin layer, a silicone layer, and a second translucent resin layer.

A resin constituting the first translucent resin layer is not particularly limited. By way of an example, the resin includes an urethane resin, a polyester resin, an acryl-based resin, a siloxane-based resin, a vinyl acetate-based resin, a styrene-based resin, a butadiene-based resin, a styrenebutadiene-based resin, a vinyl chloride-based resin, an acryl-styrene-based resin, an acryl-silicone-based resin, a polyvinyl alcohol resin, a polyether resin, an alkyd resin, polypropylene, polyvinylpyrrolidone, and the like.

A resin constituting the second translucent resin layer is not particularly limited. The resin constituting the second translucent resin layer is the same with the resin constituting the first translucent resin layer described above.

A thickness of the first translucent resin layer is not particularly limited. By way of an example, the thickness of the first translucent resin layer is preferably 0.05 $\mu$m or more, more preferably 0.1 $\mu$m or more, and further preferably 0.3 $\mu$m or more. Moreover, the thickness of the first translucent resin layer is preferably 2.0 $\mu$m or less, more preferably 1.5 $\mu$m or less, and further preferably 1.0 $\mu$m or less. The same applies to a thickness of the second translucent resin layer. When the thicknesses of the first translucent resin layer and the second translucent resin layer are within the above-described ranges, in the flake-shaped metallic color powder, a metallic color is easily expressed, and the silicone layer can be sufficiently sandwiched. Besides, a method of measuring a thickness of the first translucent resin layer is not particularly limited. By way of an example, the thickness of the first translucent resin layer can be measured by first measuring a thickness of a base material with an electronic micrometer (dial-type thickness gauge, KG3001A, manufactured by ANRITSU CORPORATION), next similarly measuring a total thickness (thickness of base material + first translucent resin layer), and calculating a difference therebetween. An example of the method of measuring the thickness of the second translucent resin layer is as follows. First, a thickness of (base material + first translucent resin layer + silicone layer) is measured with the electronic micrometer, and next a thickness of (base material + first translucent resin layer + silicone layer + second translucent resin layer) is measured with the electronic micrometer. Then, a difference between the thicknesses is calculated, so that a thickness of the second translucent resin layer can be measured.

A total light transmittance of the first translucent resin layer is not particularly limited. By way of an example, the total light transmittance is preferably 70% or more, more preferably 80% or more, and further preferably 85% or more. The same applies to a total light transmittance of the second translucent resin layer. When the total light transmittance is within the above-described ranges, in the flake-shaped metallic color powder, a metallic appearance of the silicone layer is less likely to be inhibited. Besides, in the present embodiment, the total light transmittance can be measured with NDH5000SP (manufactured by NIPPON DENSHOKU INDUSTRIES CO., LTD.).

Referring back to the general description of the flake-shaped metallic color powder, an average particle size of the flake-shaped metallic color powder is preferably 3 $\mu$m or more, more preferably 5 $\mu$m or more, further preferably 10 $\mu$m or more, and particularly preferably 15 $\mu$m or more. Moreover, the average particle size of the flake-shaped metallic color powder is preferably 900 $\mu$m or less, more preferably 600 $\mu$m or less, further preferably 450 $\mu$m or less, and particularly preferably 370 $\mu$m or less. When the average particle size is within the above-described ranges, in the flake-shaped metallic color powder, photoluminescent performance is easy to be sufficiently exhibited, and a smooth surface is easily obtained when the powder is applied.

Besides, in the present embodiment, the term "flake-shaped" refers to a shape that is a thin, a scaly, a thin plate, a flat plate, an oblate, or a flat shape. The flake-shaped powder means a powder having a substantially flat surface and a substantially uniform thickness in a direction perpendicular to the substantially flat surface. Moreover, the flake-shaped powder means a powder having a shape with a relatively small thickness and a relatively long length of the substantially flat surface. Besides, the length of the substantially flat surface is a diameter of a circle having the same projected area as a projected area of the flake-shaped powder. A shape of the substantially flat surface is not particularly limited and can be appropriately selected depending on the purpose, examples of which include polygonal shapes such as substantially rectangular, substantially square, substantially circular, substantially elliptical, substantially triangular, substantially quadrangular, substantially pentagonal, substantially hexagonal, substantially heptagonal, and substantially octagonal shapes, as well as random, indefinite shapes, etc. The average particle size can be measured by adding a flake-shaped powder into ethanol with a solid content concentration of about 0.3% by mass to 10% by mass using a laser type particle size distribution analyzer LMS-3000+MV manufactured by SEISHIN ENTERPRISE CO., LTD., and calculating a cumulative 50% volume particle size (D50).

Moreover, in the flake-shaped metallic color powder, a chroma C* value represented by the following equation (1) is preferably 1.1 or more in an L*a*b* color space. When the chroma C* is within the above-described range, the flake-shaped metallic color powder has a more excellent chroma.

$$\text{Chroma } C^* = (a^{*2}+b^{*2})^{1/2} \quad \text{Equation (1)}$$

Besides, any of "reflected light in the L*a*b* color space" can be calculated by measuring a reflected light with respect to a powder using, for example, a spectrophotometer SD7000, a control unit CU-II, and a color management software Color Mate Pro (manufactured by NIPPON DENSHOKU INDUSTRIES CO., LTD.). This case can employ a condition of a wavelength range of 380 nm to 780 nm, a light source of D65, a viewing angle of 10°, and inclusion of a regular reflection component (SCI, Specular Component Include).

In the flake-shaped metallic color powder of the present embodiment, a brightness L* value of a reflected light in the L*a*b* color space is preferably 40 or more. When the brightness L* value of the reflected light is within the above-described range, the flake-shaped metallic color powder also improves in reflected brightness (reflectance). As a result, in a laminated film, various metallic colors are easily expressed.

The flake-shaped metallic color powder preferably has a ratio of a brightness L* to a chroma C* (L*/C*) of 41 or less in the L*a*b* color space. Moreover, the ratio (L*/C*) is preferably 1 or more. When the ratio (L*/C*) is within the above-described ranges, the flake-shaped metallic color powder has a more excellent balance between the brightness and the chroma.

As described above, the flake-shaped metallic color powder of the present embodiment can realize metallic color developments of various colors in a single layer.

<Method of producing flake-shaped metallic color powder>

A method of producing a flake-shaped metallic color powder according to one embodiment of the present invention comprises a laminated body-forming step and a peeling and pulverizing step. The laminated body-forming step is a step of forming a laminated body on a base material. The laminated body-forming step comprises a preparation step of preparing a base material, a first translucent resin layer-forming step of forming a first translucent resin layer on the base material, a silicon layer-forming step of forming a silicon layer containing silicon on the first translucent resin layer, and a second translucent resin layer-forming step of forming a second translucent resin layer on the silicon layer. The peeling and pulverizing step has a peeling step of peeling the laminated body from the base material and a pulverizing step of pulverizing the peeled laminated body to obtain a flake-shaped metallic color powder. Each component will be described below.

(Laminated body-forming step)

The laminated body-forming step is a step of forming a laminated body on a base material. The laminated body-forming step comprises a preparation step of preparing a base material, a first translucent resin layer-forming step of forming a first translucent resin layer on the base material, a silicon layer-forming step of forming a silicon layer containing silicon on the first translucent resin layer, and a second translucent resin layer-forming step of forming a second translucent resin layer on the silicon layer.

Preparation step

First, a base material is prepared. The base material is not particularly limited as long as it has a smooth surface. By way of an example, the base material is a resin film, a metal foil, a composite film of a metal foil and a resin film, or the like which has flexibility, heat resistance, solvent resistance, and dimensional stability, etc. The resin film is a polyester film, a polyethylene film, a polypropylene film, a polystyrene film, a polyimide film, a polyamide film, a polycarbonate film, a triacetate film, or the like. The metal foil is a copper foil, an aluminum foil, a nickel foil, an iron foil, an alloy foil, or the like. The composite film of the metal foil and the resin film is, for example, a film obtained by laminating the above-described resin film with a metal foil.
A thickness of the base material is not particularly limited. By way of an example, the thickness of the base material is preferably 4 $\mu$m or more, more preferably 8 $\mu$m or more, and further preferably 10 $\mu$m or more. Moreover, the thickness of the base material is preferably 200 $\mu$m or less, more preferably 100 $\mu$m or less, and further preferably 50 $\mu$m or less. When the thickness of the base material is within the above-described ranges, the base material has a good handling ability and a moderate flexibility, which makes peeling, etc. easy.

First translucent resin layer-forming step

The first translucent resin-forming step is a step of forming a first translucent resin layer on a base material.
A method of forming a first translucent resin layer on a base material is not particularly limited. By way of an example, the first translucent resin layer can be formed by an inkjet method, a blade coating method, a gravure coating method, a gravure offset coating method, a bar coating method, a roll coating method, a knife coating method, an air knife coating method, a comma coating method, a U comma coating method, an AKKU coating method, a smoothing coating method, a microgravure coating method, a reverse roll coating method, a four-roll coating method, a five-roll coating method, a dip coating method, a curtain coating method, a slide coating method, a die coating method, or the like. Specifically, the first translucent resin layer is formed by gravure-coating a first translucent resin diluted with a solvent and drying it. As the solvent, an alcohol-based solvent, a ketone-based solvent, or the like can be used. Specifically, MEK, IPA, n-butanol, ethanol, or the like can be used.

Silicon layer-forming step

The silicon layer-forming step is a step of forming a silicon layer containing silicon on a first translucent resin layer.
A method of forming a silicon layer is not particularly limited. By way of an example, the silicon layer can be formed by a dry coating method such as a physical deposition method such as a vacuum deposition method, a sputtering method, and an ion plating method, which are conventionally known methods.

Second translucent resin layer-forming step

The second translucent resin layer-forming step is a step of forming a second translucent resin layer on a silicon layer.
A method of forming a second translucent resin layer on a silicon layer is not particularly limited. By way of an example, the second translucent resin layer can be formed by the same methods as those for the first translucent resin layer as described above. Specifically, the second translucent resin layer is formed by gravure-coating a second translucent resin diluted with a solvent and drying it. As the solvent, an alcohol-based solvent, a ketone-based solvent, or the like can be used. Specifically, MEK, IPA, n-butanol, ethanol, or the like can be used.
Through the steps above, a laminated body is formed in which the first translucent resin layer, the silicon layer, and the second translucent resin layer are laminated on the base material.

(Peeling and pulverizing step)

The peeling and pulverizing step has a peeling step of peeling the laminated body from the base material and a pulverizing step of pulverizing the peeled laminated body to obtain a flake-shaped metallic color powder.

Peeling step

The peeling step is a step of peeling a laminated body from a base material.

Pulverizing step

The pulverizing step is a step of pulverizing a peeled laminated body to obtain a flake-shaped metallic color powder. A method of pulverizing a laminated body is not particularly limited. By way of an example, the pulverizing method is a method using a jet mill, a ball mill, a ring roll mill, a hammer mill, a tube mill, or the like. The pulverization may be performed in water. In this case, a viscosity modifier such as alcohol, a drying accelerator, a sedimentation stabilizer, a surfactant, etc. may be used in combination. Besides, in some cases, the laminated body may become a fine flake-like metallic color powder only by peeling the laminated body from the base material, without actively performing pulverization. Moreover, coarse separation and/or classification may be performed simultaneously with pulverization.

Through the steps above, a flake-shaped metallic color powder is obtained. The flake-shaped metallic color powder can realize metallic color developments of various colors in a single layer.

<Metallic color paint>

A metallic color paint according to one embodiment of the present invention contains the above-described flake-shaped metallic color powder, resin, and a solvent. Each component will be described below.

Flake-shaped metallic color powder

A content of the flake-shaped metallic color powder is not particularly limited. By way of an example, the content of the flake-shaped metallic color powder is preferably 0.05% by mass or more, and more preferably 0.1% by mass or more, in the metallic color paint. Moreover, the content of the flake-shaped metallic color powder is preferably 10% by mass or less, and more preferably 7% by mass or less, in the metallic color paint. When the content of the flake-shaped metallic color powder is within the above-described ranges, the flake-shaped metallic color powder is easily dispersed uniformly, and there is no sedimentation or aggregation, making it easy to obtain a metallic color paint having an excellent brilliance.

Resin

Resin is not particularly limited. By way of an example, resin is an alkyd resin, a polyurethane resin, an acrylic resin, a polyvinyl chloride resin, an epoxy resin, a fluororesin or the like.

Solvent

A solvent is not particularly limited. By way of an example, the solvent is a strong solvent such as toluene, xylene, ester, and ketone, a weak solvent such as turpentine-based solvent, water or the like.

Additive

Besides, in addition to the above-described components, additives may be added to the metallic color paint as appropriate. The additives are added for the purposes of preventing sagging of a metallic color paint, preventing sedimentation during storage, preventing generation of air bubbles during painting and drying, making a surface smooth during painting and drying, etc.

The metallic color paint of the present embodiment can be prepared by mixing a flake-shaped metallic color powder, resin, and a solvent. The metallic color paint can realize metallic color developments of various colors.

<Metallic color resin pellet>

A metallic color resin pellet according to one embodiment of the present invention contains the above-described flake-shaped metallic color powder and resin. Each component will be described below.

Flake-shaped metallic color powder

A content of the flake-shaped metallic color powder is not particularly limited. By way of an example, the content of the flake-shaped metallic color powder is preferably 0.01% by mass or more in the metallic color resin pellet. Moreover, the content of the flake-shaped metallic color powder is preferably 10% by mass or less, more preferably 5% by mass or less, and further preferably 1% by mass or less, in the metallic color resin pellet. When the content of the flake-shaped metallic color powder is within the above-described ranges, a metallic color resin pellet having an excellent photoluminescent performance is easily obtained.

Resin

Resin is not particularly limited. By way of an example, resin is similar to that described above in connection with the metallic color paint.

A content of resin is not particularly limited. By way of an example, the content of resin is preferably 70% by mass or more, and more preferably 85% by mass or more, in the metallic color resin pellet. Moreover, the content of resin is preferably 99.5% by mass or less, and more preferably 99% by mass or less, in the metallic color resin pellet. When the content of resin is within the above-described ranges, a metallic color resin pellet having an excellent photoluminescent performance is easily obtained.

Additive

Besides, in addition to the above-described components, additives may be added to the metallic color resin pellet as appropriate. The additives are similar to those described above in connection with the metallic color paints.

The metallic color resin pellet of the present embodiment can be prepared by mixing a flake-shaped metallic color powder into a molten resin, cutting the mixture to have appropriate shape and size, and cooling it. The metallic color resin pellet can realize metallic color developments of various colors.

<Metallic color product>

A metallic color product according to one embodiment of the present invention contains the above-described flake-shaped metallic color powder. The metallic color product can thereby realize metallic color developments of various colors. The metallic color product is not particularly limited. By way of an example, the metallic color product is a cosmetic product, a vessel, an interior/exterior member for vehicle, or the like.

<Cosmetic product>

A cosmetic product according to one embodiment of the present invention contains the above-described flake-shaped metallic color powder. The cosmetic product can realize metallic color developments of various colors.

A method of preparing a cosmetic product is not particularly limited. By way of an example, the cosmetic product can be prepared by mixing a flake-shaped metallic color powder of the above-described embodiment into a cosmetic material such as eye shadow, foundation, manicure, and the like. The obtained cosmetic product can be thereby imparted with a glittery feel and glossiness, improving a makeup effect. Moreover, the flake-shaped metallic color powder of the present embodiment does not contain any residual formaldehyde. Therefore, the flake-shaped metallic color powder can also be used for applications in which it is applied directly to the skin. In addition, the flake-shaped metallic color powder has a small particle size and a smooth surface, so that it does not cause roughness and damage to the skin, obtaining a good feeling of use.

A content of the flake-shaped metallic color powder in the cosmetic product can be appropriately selected depending on the purpose.

<Vessel>

A vessel according to one embodiment of the present invention contains the above-described flake-shaped metallic color powder. The vessel can realize metallic color developments of various colors.

A type of the vessel is not particularly limited. By way of an example, the vessel is a tube vessel, a compact vessel, or the like for cosmetic applications. Vessels for other applications include a cup, a can, a bottle, a box, a bag, a case, a basket, a luggage, a suitcase, a container, a tank, and the like.

A method of producing a vessel is not particularly limited. By way of an example, the vessel can be made by injection molding a metallic color resin pellet. The obtained vessel can be imparted with a glittery feel and glossiness.

A content of the flake-shaped metallic color powder in the vessel can be appropriately selected depending on the purpose.

<Interior/exterior member for vehicle>

An interior/exterior member for vehicle according to one embodiment of the present invention contains the above-described flake-shaped metallic color powder. The interior/exterior member for vehicle can realize metallic color developments of various colors.

A type of the interior/exterior member for vehicle is not particularly limited. By way of an example, the interior/exterior member for vehicle includes instrument panel garnish and ornament, an audio panel, an auto air conditioner panel, a steering ornament, a door trim ornament, a power window switch bezel, an operation system knob, a switch and a cap or a variety of covers, a radiator grill, a pillar garnish, a back door ornament, a side mirror cover, an outer panel, a rear spoiler, an inside or outside door handle, a side visor, a wheel cover, a cowling for two-wheeled vehicle, and the like.

A method of producing an interior/exterior member for vehicle is not particularly limited. By way of an example, the interior/exterior member for vehicle can be produced by spray-coating a resin in which a metallic color powder is added to a target member. The obtained interior/exterior member for vehicle can be imparted with a glittery feel and glossiness.

A content of the flake-shaped metallic color powder in the interior/exterior member for vehicle can be appropriately selected depending on the purpose.

One embodiment of the present invention has been described above. The present invention is not particularly limited to the above-described embodiments. Besides, the above-described embodiment mainly describes an invention having the following configurations.

(1) A flake-shaped metallic color powder having a silicon layer containing silicon and a translucent resin layer provided on both surfaces of the silicon layer, wherein a thickness of the silicon layer is 50 nm or more.
According to such a configuration, the flake-shaped metallic color powder can realize metallic color developments of various colors in a single layer.
(2) The flake-shaped metallic color powder of (1), wherein a chroma C* value represented by the following equation (1) is 1.1 to 21 in an L*a*b* color space.

$$\text{Chroma } C^* = (a^{*2}+b^{*2})^{1/2} \quad \text{Equation (1)}$$

According to such a configuration, the flake-shaped metallic color powder has a more excellent chroma.
(3) The flake-shaped metallic color powder of (1) or (2), wherein a ratio of a brightness L* to a chroma C* (L*/C*) is 41 or less in the L*a*b* color space.
According to such a configuration, the flake-shaped metallic color powder has an excellent balance between the brightness and the chroma.
(4) A metallic color paint comprising a flake-shaped metallic color powder of any one of (1) to (3), resin, and a solvent.
According to such a configuration, the metallic color paint can realize metallic color developments of various colors.
(5) A metallic color resin pellet comprising a flake-shaped metallic color powder of any one of (1) to (3) and resin.
According to such a configuration, the metallic color resin pellet can realize metallic color developments of various colors.
(6) A cosmetic product comprising a flake-shaped metallic color powder of any one of (1) to (3).
According to such a configuration, the cosmetic product can realize metallic color developments of various colors.
(7) A vessel comprising a flake-shaped metallic color powder of any one of (1) to (3).
According to such a configuration, the vessel can realize metallic color developments of various colors.
(8) An interior/exterior member for vehicle comprising a flake-shaped metallic color powder of any one of (1) to (3).
According to such a configuration, the interior/exterior member for vehicle can realize metallic color developments of various colors.
(9) A method of producing a flake-shaped metallic color powder, the method comprising a laminated body-forming step and a peeling and pulverizing step, wherein the laminated body-forming step is a step of forming a laminated body on a base material, the step comprising a preparation step of preparing the base material, a first translucent resin layer-forming step of forming a first translucent resin layer on the base material, a silicon layer-forming step of forming a silicon layer containing silicon on the first translucent resin layer, and a second translucent resin layer-forming step of forming a second translucent resin layer on the silicon layer, and wherein the peeling and pulverizing step has a peeling step of peeling the laminated body from the base material and a pulverizing step of pulverizing the peeled laminated body to obtain a flake-shaped metallic color powder.

According to such a configuration, a flake-shaped metallic color powder can be obtained which can realize metallic color developments of various colors in a single layer.

EXAMPLE

Hereinafter, the present invention will be described in more detail with reference to Examples. The present invention is not limited to these Examples. Unless otherwise limited, "%" means "% by mass", and "part" means "part by mass".

<Preliminary study on thickness of silicon layer for expressing metallic color appearance>

A laminated body was formed on a base material by the following method, and a thickness of a silicon layer for a metallic color to express without peeling and pulverizing was studied.

(Reference example 1)

A base material (20 $\mu$m in thickness) consisting of a polyester resin was prepared (a base material preparation step). A first translucent resin was coated on the base material using a bar coater and dried to form a first translucent resin layer (1.0 $\mu$m in thickness) (a first translucent resin layer-forming step). Next, a silicon layer (36 nm in thickness) was formed on the first translucent resin layer by a vacuum deposition method (a silicon layer-forming step). Furthermore, a second translucent resin was coated on the silicon layer using the bar coater and dried to form a second translucent resin layer (1.0 $\mu$m in thickness) (a second translucent resin layer-formation step), and a vapor deposited film of Reference example 1, in which a laminated body (a first translucent resin layer/a silicon layer/a second translucent resin layer) was formed, was prepared on the base material.

(Reference examples 2 to 6)

Vapor deposited films of Reference examples 2 to 6 were prepared in the same manner as in Reference example 1, except that thicknesses of silicon layers were changed to values shown in Table 1.

The vapor deposited films of Reference examples 1 to 6 were visually evaluated to evaluate the thicknesses of the silicon layers required to express a metallic color (gold tone) from the original color tone (silver tone). The results are shown in Table 1.

Table 1

| | Film thickness (nm) of silicon layer | Metallic color appearance (visual evaluation) | Photograph (reflected light, on black sheet) |
|---|---|---|---|
| Reference example 1 | 36 | No (silver tone) | |
| Reference example 2 | 41 | No (silver tone) | |

(continued)

| | Film thickness (nm) of silicon layer | Metallic color appearance (visual evaluation) | Photograph (reflected light, on black sheet) |
|---|---|---|---|
| Reference example 3 | 47 | No (silver tone) | |
| Reference example 4 | 58 | Yes (gold tone) | |
| Reference example 5 | 64 | Yes (gold tone) | |
| Reference example 6 | 70 | Yes (gold tone) | |

As shown in Table 1, it was confirmed that, when a thickness of a silicon layer was 50 nm or more (Reference examples 4 to 6), a metallic color (gold tone) was expressed from the original color tone (silver tone). In addition, it was confirmed that, even in a case where a base material was peeled off from the above-described vapor deposited film and pulverized to prepare a flake-shaped metallic color powder, when the thickness of the silicon layer was 50 nm or more, a metallic color (gold tone) was expressed from the original color tone (silver tone).

<Example 1>

A base material (20 μm in thickness) consisting of a polyester resin was prepared (a base material preparation step). A first translucent resin was coated on the base material using a bar coater and dried to form a first translucent resin layer (1.0 μm in thickness) (a first translucent resin layer-forming step). Next, a silicon layer (50 nm in thickness) was formed on the first translucent resin layer by a vacuum deposition method (a silicon layer-forming step). Furthermore, a second translucent resin was coated on the silicon layer using the bar coater and dried to form a second translucent resin layer (1.0 μm in thickness) (a second translucent resin layer-formation step), and a vapor deposited film, in which a laminated body (a first translucent resin layer/a silicon layer/a second translucent resin layer) was formed, was prepared on the base material. The base material was peeled off from the obtained vapor deposited film (peeling step). Next, the laminated body was pulverized with a ball mill to obtain a flake-shaped metallic color powder (dimension: 30 μm).

<Examples 2 to 8, Comparative examples 1 and 2>

A flake-shaped metallic color powder was obtained in the same manner as in Example 1, except that the formulation was changed to that shown in Table 2.

A color tone of the obtained flake-shaped metallic color powder was visually observed, and optical properties were measured under the following condition. The results are shown in Table 2. Besides, a chroma C* and a hue angle h were calculated according to the following equation.

(Calculating equation of chroma C*)

$$\text{Chroma } C^* = (a^{*2}+b^{*2})^{1/2}$$

(Calculating equation of hue angle h)

$$\text{Hue angle } h = \tan^{-1}(b^*/a^*) \text{ (unit:°)}$$

(Distance from origin in L*a*b* color space)

$$\text{Distance from origin} = (L^{*2}+a^{*2}+b^{*2})^{1/2}$$

(Measuring device, measurement condition)

Spectrophotometer SD7000, a control unit CU-II, a color management software Color Mate Pro
Manufactured by NIPPON DENSHOKU INDUSTRIES CO., LTD.
Measuring a reflected light with respect to a powder

·Wavelength range: 380 nm to 780 nm
·Light source: D65
·Viewing angle: 10°
·Inclusion of a regular reflection component (SCI, Specular Component Include)

Table 2

| | Material of intermediate layer | Film thickness of intermediate layer (nm) | Appearance (visual evaluation) | | CIE 1976 (L*a*b*) color space | | |
|---|---|---|---|---|---|---|---|
| | | | Metallic color appearance | Reflected color | L* | a* | b* |
| Comparative example 1 | Al | 25 | No | Silver | 75.69 | 0.48 | 1.30 |
| Comparative example 2 | Al | 40 | No | Silver | 88.12 | 0.50 | -0.47 |
| Example 1 | Si | 50 | Yes | Light yellow | 71.73 | 4.22 | 15.33 |
| Example 2 | Si | 60 | Yes | Yellow | 67.54 | 4.78 | 18.09 |
| Example 3 | Si | 70 | Yes | Orange | 59.77 | 11.78 | 16.89 |
| Example 4 | Si | 80 | Yes | Pink | 52.91 | 14.42 | 7.07 |
| Example 5 | Si | 90 | Yes | Purple | 50.48 | 7.82 | 2.92 |
| Example 6 | Si | 100 | Yes | Blue | 46.91 | -0.86 | -0.76 |
| Example 7 | Si | 110 | Yes | Green | 47.46 | -0.22 | 3.95 |
| Example 8 | Si | 120 | Yes | Brown | 48.00 | 7.44 | 4.78 |

(continued)

| | Chroma | Hue angle | L*/C* | L*/film thickness | Distance from origin in L*a*b* color space |
|---|---|---|---|---|---|
| | C* | h | | | |
| Comparative example 1 | 1.39 | 69.73 | 54.62 | 3.03 | 75.70 |
| Comparative example 2 | 0.69 | -43.23 | 128.41 | 2.20 | 88.12 |
| Example 1 | 15.90 | 74.61 | 4.51 | 1.43 | 73.47 |
| Example 2 | 18.71 | 75.20 | 3.61 | 1.13 | 70.08 |
| Example 3 | 20.59 | 55.11 | 2.90 | 0.85 | 63.22 |
| Example 4 | 16.06 | 26.11 | 3.29 | 0.66 | 55.29 |
| Example 5 | 8.35 | 20.48 | 6.05 | 0.56 | 51.16 |
| Example 6 | 1.15 | 41.47 | 40.87 | 0.47 | 46.92 |
| Example 7 | 3.96 | -86.77 | 11.99 | 0.43 | 47.62 |
| Example 8 | 8.84 | 32.75 | 5.43 | 0.40 | 48.80 |

As shown in Table 2, the flake-shaped metallic color powder of the present invention was able to realize metallic color developments of various colors in a single layer.

**Claims**

1.  A flake-shaped metallic color powder having a silicon layer containing silicon and a translucent resin layer provided on both surfaces of the silicon layer,
    wherein a thickness of the silicon layer is 50 nm or more.

2.  The flake-shaped metallic color powder of claim 1, wherein a chroma C* value represented by the following equation (1) is 1.1 to 21 in an L*a*b* color space,

$$\text{Chroma } C^* = (a^{*2}+b^{*2})^{1/2} \quad \text{Equation (1)}$$

3.  The flake-shaped metallic color powder of claim 1 or 2, wherein a ratio of a brightness L* to a chroma C* (L*/C*) is 41 or less in the L*a*b* color space.

4.  A metallic color paint comprising a flake-shaped metallic color powder of any one of claim 1 or 2, resin, and a solvent.

5.  A metallic color resin pellet comprising a flake-shaped metallic color powder of any one of claims 1 to 3 and resin.

6.  A metallic color product comprising a flake-shaped metallic color powder of any one of claims 1 to 3.

7.  A method of producing a flake-shaped metallic color powder, the method comprising a laminated body-forming step and a peeling and pulverizing step,

    wherein the laminated body-forming step is a step of forming a laminated body on a base material, the step comprising:

    a preparation step of preparing the base material;
    a first translucent resin layer-forming step of forming a first translucent resin layer on the base material;
    a silicon layer-forming step of forming a silicon layer containing silicon on the first translucent resin layer; and
    a second translucent resin layer-forming step of forming a second translucent resin layer on the silicon layer; and

wherein the peeling and pulverizing step has:

a peeling step of peeling the laminated body from the base material; and
a pulverizing step of pulverizing the peeled laminated body to obtain a flake-shaped metallic color powder.

## INTERNATIONAL SEARCH REPORT

| | International application No. |
|---|---|
| | **PCT/JP2023/011493** |

| A. | CLASSIFICATION OF SUBJECT MATTER |
|---|---|
| | ***C01B 33/02***(2006.01)i; ***A61K 8/85***(2006.01)i; ***C09C 1/28***(2006.01)i; ***C09C 1/62***(2006.01)i<br>FI:  C09C1/28; C01B33/02 D; A61K8/85; C09C1/62 |

According to International Patent Classification (IPC) or to both national classification and IPC

| B. | FIELDS SEARCHED |
|---|---|

Minimum documentation searched (classification system followed by classification symbols)

      C01B33/02; A61K8/85; C09C1/28; C09C1/62

Documentation searched other than minimum documentation to the extent that such documents are included in the fields searched

      Published examined utility model applications of Japan 1922-1996
      Published unexamined utility model applications of Japan 1971-2023
      Registered utility model specifications of Japan 1996-2023
      Published registered utility model applications of Japan 1994-2023

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | JP 2013-093131 A (OIKE IND. CO., LTD.) 16 May 2013 (2013-05-16)<br>   entire text | 1-7 |
| A | WO 2021/260518 A1 (ECKART AMERICA CORP.) 30 December 2021 (2021-12-30)<br>   entire text | 1-7 |
| A | JP 2022-013101 A (NIPPON PAINT AUTOMOTIVE COATINGS CO., LTD.) 18 January<br>2022 (2022-01-18)<br>   entire text | 1-7 |
| A | JP 2002-500258 A (FLEX PRODUCTS INC.) 08 January 2002 (2002-01-08)<br>   entire text | 1-7 |
| A | JP 2004-124069 A (SHOWA DENKO KK) 22 April 2004 (2004-04-22)<br>   entire text | 1-7 |

☐ Further documents are listed in the continuation of Box C.   ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **26 May 2023** | **06 June 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

INTERNATIONAL SEARCH REPORT
Information on patent family members

International application No.

**PCT/JP2023/011493**

| Patent document cited in search report | | | Publication date (day/month/year) | Patent family member(s) | | | Publication date (day/month/year) |
|---|---|---|---|---|---|---|---|
| JP | 2013-093131 | A | 16 May 2013 | (Family: none) | | | |
| WO | 2021/260518 | A1 | 30 December 2021 | (Family: none) | | | |
| JP | 2022-013101 | A | 18 January 2022 | WO | 2022/004469 | A1 | |
| JP | 2002-500258 | A | 08 January 2002 | US | 6013370 | A | |
| | | | | entire text | | | |
| | | | | WO | 1999/035194 | A1 | |
| | | | | EP | 1044243 | A1 | |
| | | | | AU | 1387999 | A | |
| | | | | CA | 2315845 | A | |
| | | | | CN | 1280598 | A | |
| | | | | KR | 10-0537825 | B1 | |
| JP | 2004-124069 | A | 22 April 2004 | (Family: none) | | | |

Form PCT/ISA/210 (patent family annex) (January 2015)

**EP 4 477 621 A1**

**Patent documents cited in the description**

- JP 2015532676 A